(19) Europäisches Patentamt
European Patent Office
Office européen des brevets

(11) **EP 0 819 938 B1**

(12) **EUROPÄISCHE PATENTSCHRIFT**

(45) Veröffentlichungstag und Bekanntmachung des Hinweises auf die Patenterteilung:
**26.03.2008   Patentblatt 2008/13**

(51) Int Cl.:
*G01N 33/28* (2006.01)        *G01N 27/08* (2006.01)
*G01N 27/10* (2006.01)

(21) Anmeldenummer: **97110884.0**

(22) Anmeldetag: **02.07.1997**

(54) **Verfahren und Messsysteme zur Messung physikalischer Grössen von gering leitenden und nichtleitenden Fluiden**

Methods and systems for measuring physical quantities of poorly conductive and non-conductive fluids

Méthodes et systèmes pour la mesure de grandeurs physiques de fluides peu conductives et non-conductives

(84) Benannte Vertragsstaaten:
**DE FR GB IT**

(30) Priorität: **17.07.1996  DE 19628690**

(43) Veröffentlichungstag der Anmeldung:
**21.01.1998   Patentblatt 1998/04**

(73) Patentinhaber: **ACHENBACH BUSCHHÜTTEN GmbH**
**57223 Kreuztal (DE)**

(72) Erfinder:
• **Kuipers, Ulrich, Prof. Dr.-Ing.**
**57462 Olpe (DE)**

• **Barten, Axel, Dipl.-Ing.**
**57078 Siegen (DE)**
• **Kohlrausch, Arnt, Dipl.-Ing.**
**57271 Hilchenbach (DE)**

(74) Vertreter: **Pürckhauer, Rolf**
**Am Rosenwald 25**
**57234 Wilnsdorf (DE)**

(56) Entgegenhaltungen:
WO-A-96/07094          SU-A- 1 044 893
US-A- 4 646 070         US-A- 5 033 293
US-A- 5 124 654         US-A- 5 315 243
US-A- 5 399 979

EP 0 819 938 B1

Printed by Jouve, 75001 PARIS (FR)

## Beschreibung

Die Erfindung betrifft ein Verfahren und ein Messsystem zur Online-Messung physikalischer Größen von gering leitenden und nichtleitenden Fluiden, wobei sich das Fluid zwischen zwei elektrisch leitenden Elektroden eines Basissensors befindet.

Im Bereich des Walzölsystems des Hauptbehälters von Walzanlagen sowie im Bereich der Walzgerüste treten von Zeit zu Zeit Verpuffungen und Brände auf, die durch elektrostatische Entladungen ausgelöst werden.

Eine Ausbildung von elektrostatischen Ladungen aufgrund von Reibungseffekten tritt im Walzöl trotz einer Erdung der Walzgerüste und aller beteiligten Komponenten auf, wenn die elektrische Leitfähigkeit des Öls unter einen Mindestwert fällt, so dass ein Ausgleich der elektrischen Ladungen nicht mehr gewährleistet ist.

Um der Gefahr von elektrostatischen Entladungsvorgängen und der damit verbundenen Brandgefahr in Walzwerken entgegenzuwirken, sollte die elektrische Leitfähigkeit des Walzöls laufend überprüft werden. Bei Unterschreitung eines bestimmten Wertes kann man dem Öl ggf. Leitfähigkeitsadditive beimischen.

Auf dem Markt sind Messgeräte erhältlich, mit denen man nach verschiedenen Verfahren die elektrische Leitfähigkeit von Fluiden bestimmen kann. Vorhandene Leitfähigkeitsmessgeräte für nicht- oder geringleitende Fluide wie z.B. Öle oder Kraftstoffe sind zur Messung in strömenden Medien (Online-Betrieb) nicht geeignet. Das zu messende Fluid muss sich bei den bekannten Systemen mehrere Minuten im Ruhezustand befinden, damit das Messgerät einen verlässlichen Wert anzeigt. Die laufende Entnahme von Messproben ist zeit- und arbeitsaufwendig und mit entsprechenden Kosten verbunden. Ein weiterer Nachteil der Offline-Messgeräte ist ferner darin zu sehen, dass man nur begrenzt verlässliche Messwerte der elektrischen Leitfähigkeit erlangen kann, da sich die Leitfähigkeit binnen kurzer Zeit durch viele Einflüsse ändern kann. Bei der Messung eines im Gefahrenbereich liegenden Wertes ist man bestrebt, durch Zugabe von Additiven z.B. in Walzöl diesen wieder zu senken, damit eine reibungslose Produktion gewährleistet werden kann. Da zwischen Probeentnahme und Messzeitpunkt, bedingt durch die vom Messgerätehersteller vorgeschriebene Ruhrzeit des Öles, nennenswerte Zeitspannen liegen, kann es unter Umständen zur Abstellung der Gefahr elektrostatischer Entladungen bereits zu spät sein.

Dies gilt in besonderem Maße für einen mechanischen Filtervorgang, bei dem aus dem Walzöl der Abrieb des gewalzten Materials herausgefiltert wird und durch das Zerteilen und Verästeln des Ölstromes über die Poren und feinen Kapillaren eines Filtermittels wie Kieselgur große elektrische Ladungsmengen durch Ladungstrennung oder Reibungselektrizität erzeugt werden.

Ein entscheidender Nachteil der auf dem Markt befindlichen Messgeräte zur Messung der elektrischen Leitfähigkeit von gering leitenden und nichtleitenden Fluiden ist darin zu sehen, dass unvermeidbare parasitäre Kapazitäten der bei den Messgeräten verwendete Sensoren, ebenso wie Polarisationskapazitäten, große Messabweichungen bewirken.

Zur Messung von Verschmutzungen von Fluiden sind optische Messgeräte auf dem Markt, mit denen die Trübung zur Bestimmung des Verschmutzungsgrades der Fluide gemessen wird. Zwischenzeitlich hat man jedoch festgestellt, dass die Trübung kein Maß für den Verschmutzungsgrad von vielen Fluiden wie z.B. Ölen sein kann, da die geometrischen Abmessungen der im Fluid enthaltenen Schmutzpartikel zum Teil kleiner sind als die Wellenlänge des Lichtes, so dass diese kleinsten Schmutzpartikel durch eine auf einer Brechung des Lichtes basierenden Trübungsmessung nicht mehr erfasst werden.

Die fehlerhafte Funktionsweise der auf dem Markt befindlichen Geräte zur Messung der elektrischen Leitfähigkeit und des Verschmutzungsgrades von Ölen, Kraftstoffen und dgl. nichtleitenden und gering leitenden Fluiden führt dazu, dass Mittel zur Erhöhung der elektrischen Leitfähigkeit von Fluiden wie Walzöl diesen prophylaktisch beigegeben werden und dass beim Filtern von Ölen Filtermittel wie Kieselgur und Bleicherde weit vor oder erst nach ihrer Erschöpfung ausgetauscht werden. Bekannte, auch als Leitfähigkeitsadditive bezeichnete Mittel zur Erhöhung der Leitfähigkeit sind ökologisch und gesundheitlich bedenklich, so dass eine pauschale, vorbeugende Beimischung dieser Mittel hohe Umweltbelastungen und entsorgungstechnische Schwierigkeiten verursacht. Durch eine aufgrund eines verstärkten Einsatzes von Filtermitteln bewirkte hohe Filtratqualität und durch Reaktionen der Leitfähigkeitsadditive mit den Filtermitteln kann die Leitfähigkeit des Öles und der Ölerzeugnisse stark vermindert werden, und infolge der verminderten Leitfähigkeit entstehen hohe elektrische Aufladungen, die zu unkontrollierten Verpuffungen und dem Brand von Maschinen und Anlagen führen können.

Die US-A-5124654 beschreibt ein Messsystem zur Feststellung des Alkoholgehaltes und/oder des Heizwertes von Kraftstoffen durch eine Online-Messung physikalischer Größen wie der Dielektrizitätskonstanten und der elektrischen Leitfähigkeit. Die Messeinrichtung des Messsystems besteht im wesentlichen aus dem vor den Kraftstoffeinspritzventilen einer Einspritzbrennkraftmaschine angeordneten Kraftstoffverteilerrohr, das als Teil des Kraftstoffsystems die äußere Elektrode eines Kondensators zur Messung der Dielektrizitätskonstanten des Kraftstoffs bildet. Innerhalb des aus einem elektrisch leitfähigen Material hergestellten Verteilerrohrs befindet sich eine innere zylindrische Gegenelektrode, die durch Abstandshalter in einem konstanten Abstand zu dem als Außenelektrode wirkenden Verteilerrohr gehalten wird. Der elektrische Anschluss der Innenelektrode zur Messung der Dielektrizitätskonstanten ist durch eine Verschraubung mit Isolation nach außen geführt und kann unmittelbar mit einer elektronischen Messschaltung verbunden werden. Eine zusätzliche Innenelektrode, die koaxial zu der erstgenannten Innenelektrode angeordnet und von dieser durch eine Isolation getrennt ist, dient zusammen mit der Außenelektrode zur Messung der elektrischen Leitfähigkeit des Kraftstoffs.

Eine ähnliche Messeinrichtung, die zwei Platten eines Kondensators zur Messung der Dielektrizitätskonstanten eines Fluids umfasst, ist aus der US-A-5399979 bekannt.

Die in der US-A-5124654 beschriebene Messeinrichtung weist den Nachteil auf, dass bei einer Ablagerung von Schmutzpartikeln auf den Halterungen der Innenelektrode Fehler bei den Messsignalen auftreten.

Aus der SU-A-1 044 893 ist ein Verfahren zur automatischen Kontrolle des technischen Zustandes von Verbrennungsmotoren bekannt. Bei diesem Verfahren werden periodische Probennahmen vor und hinter dem Ölfilter des Motors mit Hilfe von Sensoren durchgeführt. Die Sensoren liefern elektrische Signale, die proportional zur Konzentration von Verschleißprodukten im Öl sein sollen. Die Funktion der Sensoren wird nicht beschrieben.

Der Erfindung liegt die Aufgabe zugrunde, ein für gering leitende und nichtleitende Fluide wie Walzöl, Schmieröl, Hydrauliköl, Bremsflüssigkeiten und Kraftstoffe bestimmtes Verfahren und ein Messsystem zur Online-Messung der elektrischen Leitfähigkeit zur Regelung einer elektrostatische Auf- und Entladungen verhindernden Mindestleitfähigkeit durch eine geregelte Einleitung von Leitfähigkeitsadditiven, zur Online-Messung der zeitlichen Änderung der Leitfähigkeit zur Bestimmung des Feinstverschmutzungseintrags und des Verunreinigungsgrades sowie zur Differenzmessung der Leitfähigkeit vor und hinter einem Filter zur Prüfung der Funktion und Güte eines Filtervorganges und gegebenenfalls zur Regelung des Filtervorganges durch eine geregelte Zuführung von Filterhilfsmitteln zu entwickeln.

Diese Aufgabe ist erfindungsgemäß gelöst durch das Messverfahren nach Patentanspruch 1 sowie das Messsystem nach Patentanspruch 7.

Das erfindungsgemäße Verfahren und das Messsystem zur Messung der elektrischen Leitfähigkeit von gering leitenden und nichtleitenden Fluiden wie Walzöl sowie die Vorteile des Verfahrens und des Messsystems gegenüber dem Stand der Technik sind nachstehend anhand schematischer Zeichnungen erläutert, die im einzelnen folgendes darstellen:

Fig. 1      eine schematische Darstellung eines Walzölkreislaufes mit einem Messsystem zur Bestimmung der Leitfähigkeit des Walzöls,

Fig. 2      eine schematische Längsschnittdarstellung eines Basissensors,

Fig. 3      das Ersatzschaltbild des Basissensors nach Fig. 2, übernommen aus: Oehme, F.: Chemische Sensoren, Vieweg 1991,

Fig. 4      das vereinfachte Ersatzschaltbild des Basissensors,

Fig. 5      einen schematischen Querschnitt des Basissensors nach Fig. 2,

Fig. 6      das Blockschaltbild eines Messsystems mit dem Basissensor nach den Fign. 2 bis 5 zur Messung verschiedener physikalischer Größen von gering leitenden und nichtleitenden Fluiden,

Fig. 7      das Schaltbild eines mit einer Gleichspannung betriebenen Basissensors zur Messung des ohmschen Widerstandes eines Fluids,

Fig. 8      den Verlauf einer bipolar getakteten Gleichspannung zum Betreiben eines Basissensors zur Messung des ohmschen Widerstands eines Fluids,

Fig. 9      das Blockschild eines Messsystems zur Messung der elektrischen Leitfähigkeit eines Fluids,

Fig. 10     das Schaltbild des bei dem Messsystem nach Fig. 9 eingesetzten Integrators mit Sensor und Analogschalter,

Fig. 11     das Diagramm der getakteten Eingangsspannung und der Ausgangsspannung des Integrators und

Fig. 12     das Blockschaltbild eines Messsystems zur Messung der elektrischen Leitfähigkeit und der Dielektrizitätszahl eines Fluids.

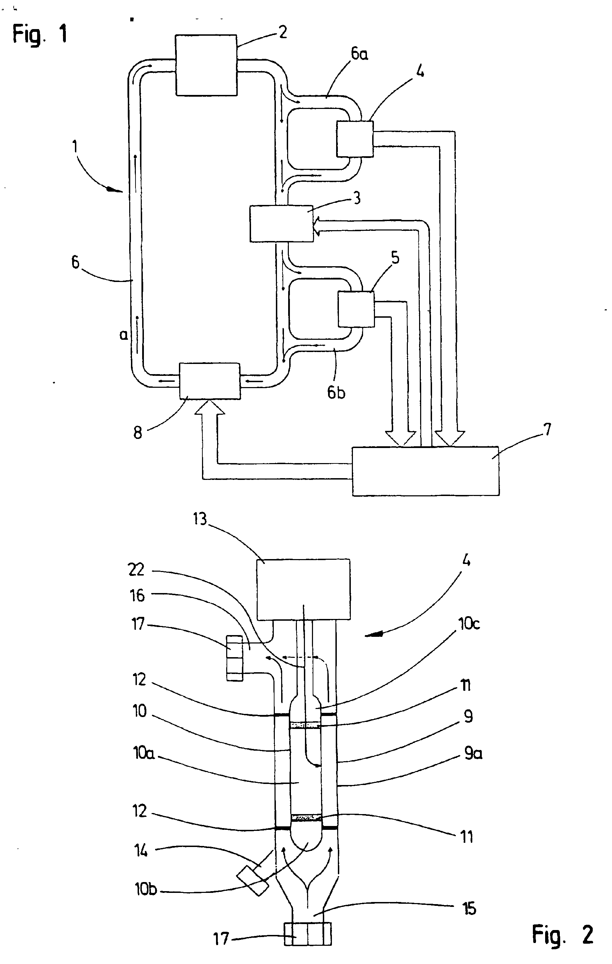

Der in Fig. 1 schematisch dargestellte Walzölkreislauf 1 eines Kaltbandwalzwerkes 2 mit einem Schmier- und Kühlöl für den Walzvorgang ist mit einem Messsystem zur Online-Messung der elektrischen Leitfähigkeit des Walzöls, des Grades der Ölverschmutzung, die hauptsächlich durch den Abrieb der Walzen und des Walzgutes sowie Additive bewirkt wird, sowie der Qualität des Filterprozesses eines in dem Kreislauf angeordneten Ölfilters 3 ausgerüstet.

Das Messsystem umfasst zwei gleiche Basissensoren 4, 5. Der Basissensor 4 ist - in Umlaufrichtung a des Walzöls gesehen - in einer Bypassleitung 6a der Hauptleitung 6 des Walzölkreislaufs 1 vor dem Ölfilter 3 und der Basissensor 5 in einer Bypassleitung 6b der Hauptleitung 6 hinter dem Ölfilter 3 eingebaut.

Mit dem hinter dem Ölfilter 3 in der Bypassleitung 6b eingebauten Basissensor 5 wird laufend der Absolutwert der elektrischen Leitfähigkeit des Walzöls zur Ermittlung des Grades der elektrostatischen Aufladung im Hinblick auf die Vermeidung elektrostatischer Entladungen gemessen. Die Messsignale des Basissensors 5 werden einer Regeleinrichtung 7 übermittelt, die eine Vorrichtung 8 zur Einleitung von Leitfähigkeitsadditiven in den Walzölkreislauf 1 regelt. Durch die Zugabe von Leitfähigkeitsadditiven wird die geforderte Mindestleitfähigkeit des Walzöls von 50 pS/m eingeregelt.

Der durch den Basissensor 5 hinter dem Ölfilter 3 gemessene Absolutwert der elektrischen Leitfähigkeit des Walzöls, der ggf. auf die Öltemperatur bezogen werden muss, ist ferner ein Kriterium für den Verschmutzungsgrad des Öls und damit ausschlaggebend für den Zeitpunkt einer Aufbereitung des verbrauchten und durch einen Filterprozess nicht mehr zu reinigenden Walsöls durch einen Destillations- bzw. Rektifizierprozess.

Mittels der Differenzwerte, die sich aus den von dem Basissensor 4 vor dem Ölfilter 3 gemessenen Werten der elektrischen Leitfähigkeit des Walzöls und den von dem Basissensor 5 hinter dem Ölfilter 3 gemessenen Werten der Leitfähigkeit ergeben, wird die Qualität des Filterprozesses bewertet und der Zeitpunkt der erforderlichen Reinigung des Filters bzw. der Erneuerung des Filtermittels festgelegt und die Zufuhr von Filterhilfsmitteln geregelt.

Der zur Messung der elektrischen Leitfähigkeit $\kappa$ von gering leitenden und nichtleitenden Fluiden wie Walzöl einsetzbare Basissensor 4 nach Fig. 2 ist in senkrechter Lage in die Bypassleitung 6a der Hauptleitung 6 des Walzölkreislaufes 1 eingebaut, um zu vermeiden, dass sich im Ölkreislauf befindliche Luft im Basissensor ansammelt, die die Messergebnisse verfälschen würde. Der Basissensor 4 weist eine Außenelektrode 9 auf, die als ein elektrisch leitender metallischer Rohrabschnitt 9a ausgebildet ist, in dem eine elektrisch leitende, vom Fluid umströmte, vorzugsweise stromlinienförmig ausgebildete Innenelektrode 10 angeordnet ist. Die zylindrische Innenelektrode 10 ist in eine mittlere Nutzelektrode 10a sowie eine vordere und eine hintere Schirmelektrode 10b, 10c aufgeteilt. Die Nutzelektrode 10a der Innenelektrode 10 ist durch Isolierungen 11 von den beiden Schirmelektroden 10b, 10c getrennt. Die Innenelektrode 10 ist durch zwei Distanzhalter 12 in dem Basissensor 4 gehalten, wobei die Distanzhalter 12 zwischen der Außenelektrode 9 und den beiden Schirmelektroden 10b, 10c eingebaut sind. Durch diese Anordnung werden inhomogene Randfelder vermieden, die bei einer Ablagerung von elektrisch leitenden Schmutzpartikeln an den Distanzhaltern 12 zu falschen Messergebnissen führen. Die Innenelektrode 10 ist zur Durchführung von Anschlussleitungen für die Messelektronik 13 teilweise als Hohlkörper ausgebildet.

Ein in die Außenelektrode 9 des Basissensors 4 eingebauter Temperatursensor 14 dient zur Messung der Temperatur des Walzöls.

Zur Messung der Viskosität des Walzöls kann die Innenelektrode 10 des Basissensors 4 mit einem Drehantrieb ausgestattet werden, dessen Energieverbrauch oder Rotationsgeschwindigkeit eine Messgröße für die Viskosität des Walzöls darstellt.

Die rohrförmige Außenelektrode 9 weist einen Eintritts- und einen Austrittsstutzen 15, 16 jeweils mit einem Rohranschluss 17 für den Einbau des Basissensors 4 in die Bypassleitung 6a der Hauptleitung 6 des Walzölkreislaufs 1 auf.

Zur Bestimmung der elektrischen Leitfähigkeit $\kappa$ des Walzöls wird der ohmsche Widerstand $R_x$ des Öls zwischen der Außenelektrode 9 und der Innenelektrode 10 des Basissensors 4 mit dem nachfolgend beschriebenen Messsystem gemessen.

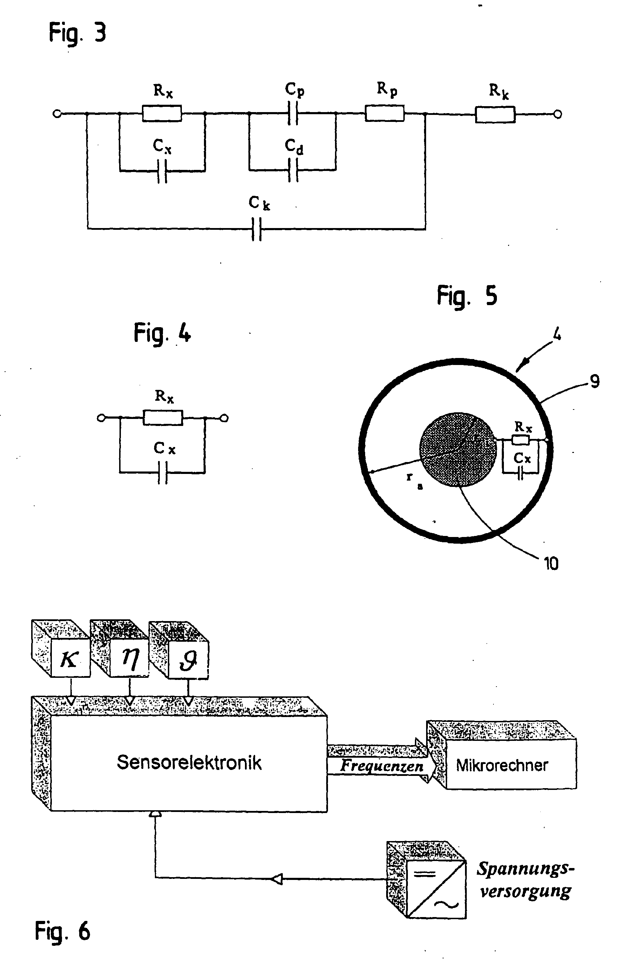

Das Ersatzschaltbild des Gesamtsensors nach Fig. 3 zeigt, dass der gesuchte Widerstand $R_x$ Bestandteil eines Netzwerks von parasitären Widerständen und Kondensatoren ist. In dem Ersatzschaltbild bedeuten:

$R_x$     gesuchter Widerstand
$C_x$     Kapazität des Sensors
$R_p$     Polarisationswiderstand
$C_p$     Polarisationskapazität
$C_d$     Doppelschichtkapazität
$C_k$     Kabelkapazität
$C_k$     Kabel- und Kontaktwiderstand

Dieses Ersatzschaltbild lässt sich bei geeigneter Sensorauslegung und unter Verwendung eines geeigneten Sensorsignals erheblich vereinfachen. So können $C_k$ und $R_k$ vernachlässigt werden, wenn die Anschlüsse zur Innen- bzw. Außenelektrode getrennt verlegt werden, der Anschluss der Innenelektrode über ein Koaxialkabel erfolgt und der Außenleiter des Koaxialkabels geeignet angeschlossen wird.

Die Leitfähigkeit nimmt bei Anlegen einer Gleichspannung ab. Dies ist auf die Bildung von $C_p$ $C_d$ und $R_k$

zurückzuführen. Wird ein Messsignal mit wechselnder Polarität verwendet, so sind auch diese Komponenten im Ersatzschild zu vernachlässigen. Hierdurch ergibt sich das endgültige Ersatzschaltbild des Basissensors nach Fig. 4.

Mit Hilfe der Grundformeln für homogene Strömungsfelder lassen sich bei einer vorgegebenen Sensorgeometrie der Widerstand $R_x$ bzw. die spezifische Leitfähigkeit $\kappa$ des in Fig. 5 im Querschnitt dargestellten Sensors 4 mit einer koaxialen Anordnung der Außenelektrode 9 und der Innenelektrode 10 in Form eines Einschichtzylinderkondensators wie folgt berechnen:

$$R_X = \frac{\ln\left(\dfrac{r_a}{r_i}\right)}{2\pi l \kappa} \qquad\qquad (1)$$

Es bedeuten:

$r_a$   Innenradius der Außenelektrode
$r_i$   Radius der Innenelektrode
l    aktive Elektrodenlänge

Fig. 6 zeigt das Blockschaltschild des neuen Messsystems mit dem vorbeschriebenen Basissensor zur Messung der spezifischen Leitfähigkeit $\kappa$, der Viskosität $\eta$ und der Temperatur $\vartheta$ von gering leitenden und nichtleitenden Fluiden wie Walzöl.

Nachfolgend ist das erfindungsgemäße Messsystem mit der zugehörigen Elektronik zur Bestimmung des gesuchten Widerstandes $R_x$ und der parasitären Sensorkapazität $C_x$ erläutert. Sind der Widerstand $R_x$ und die Sensorkapazität $C_x$ bekannt, kann die spezifische Leitfähigkeit $\kappa$ berechnet werden. Da diese beiden Größen temperaturabhängig sind, ist eine Temperaturmessung erforderlich, die nicht näher erläutert ist.

Zur Bestimmung des Widerstandes $R_x$ und der parasitären Sensorkapazität $C_x$ werden integrierende Messsystems benutzt. Diese Messsysteme zeichnen sich durch eine hohe Störunterdrückung aus und bieten den weiteren Vorteil, dass die mit Hilfe der Integration gewonnenen Messgrößen leicht in Frequenzen umgewandelt werden können, die sehr genau messbar sind. Weitere vorteilhafte Eigenschaften von Frequenzen sind eine störungsfreie Fernübertragung des Messsignals und eine einfache Digitalisierung. Dies ist von entscheidender Bedeutung, da die zu bestimmenden Größen $R_x$ und $C_x$ mit einem Mikrorechner erfasst werden, wie dies aus dem Blockschaltbild des Meßsystems nach Fig. 6 hervorgeht. Bei diesem Messsystem ist die Spannungsversorgung getrennt angeordnet, um durch diese verursachte Störungen auf die Messelektronik auszuschließen.

Das Ersatzschaltbild des Leitfähigkeitssensor reduziert sich auf die Parallelschaltung eines Widerstandes $R_x$ und einer Kapazität $C_x$. Zur Bestimmung der Leitfähigkeit ist jedoch nur der Widerstand $R_x$ relevant, d.h. die parasitäre Sensorkapazität $C_x$ soll nicht in das Messergebnis eingehen. Eine Möglichkeit, die Kapazität zu eliminieren, besteht darin, mit einer Gleichspannung zu messen. Für den aufgeladenen Kondensator gilt dann nach

$$i_C = C \cdot \frac{du\,(t)}{dt} \qquad \text{mit } \frac{du}{dt} = 0 \;:\; i_C = 0 \qquad (2)$$

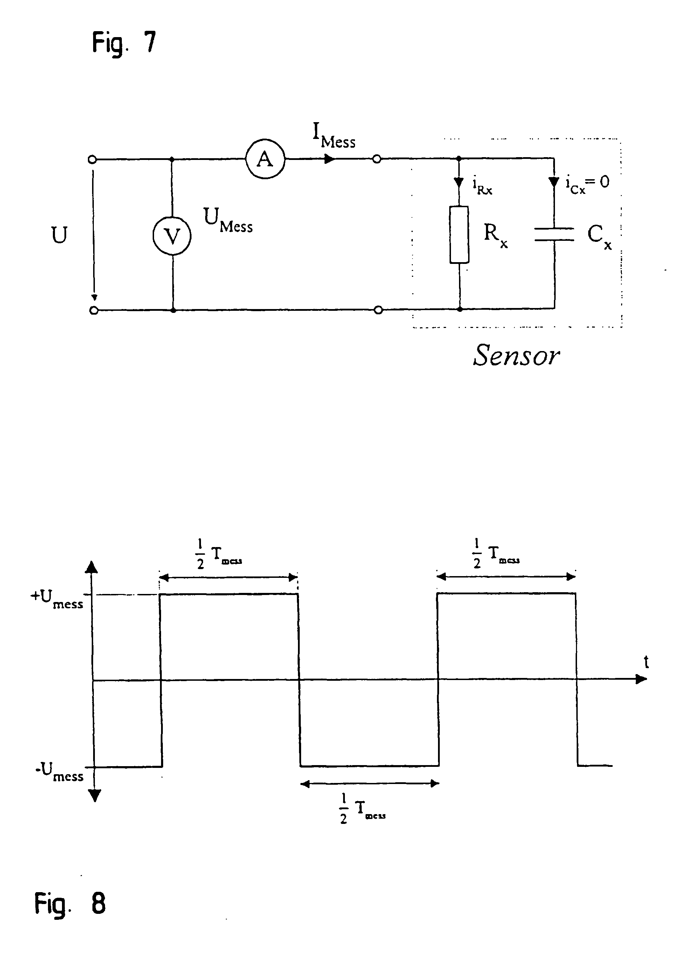

Das Messsystem nach Fig. 7 kompensiert nun zwar die Sensorkapazität $C_x$., lässt aber gleichzeitig andere Störgrößen wirksam werden. Vor allem eine auftretende Polarisation macht eine Widerstandsbestimmung über eine Gleichspannungsmessung unmöglich. Auch würden Offsetgrößen der Operationverstärker eine weitere Verfälschung des Messergebnisses bewirken. Um diese zu verhindert, ist es unabdingbar, mit einer Wechselspannung zu arbeiten. Da aber eine reine Sinusspannung den Blindstrom der Sensorkapazität nicht kompensiert, wird diese mitgemessen. Das erfindungsgemäße Verfahren arbeitet mit einer bipolar getakteten Gleichspannung, wobei aber nur in den Zeitabschnitten gemessen wird, in denen die am Sensor anliegende Spannung konstant ist.

Fig. 8 zeigt den Verlauf der bipolar getakteten Gleichspannung mit den eingezeichneten Messintervallen.

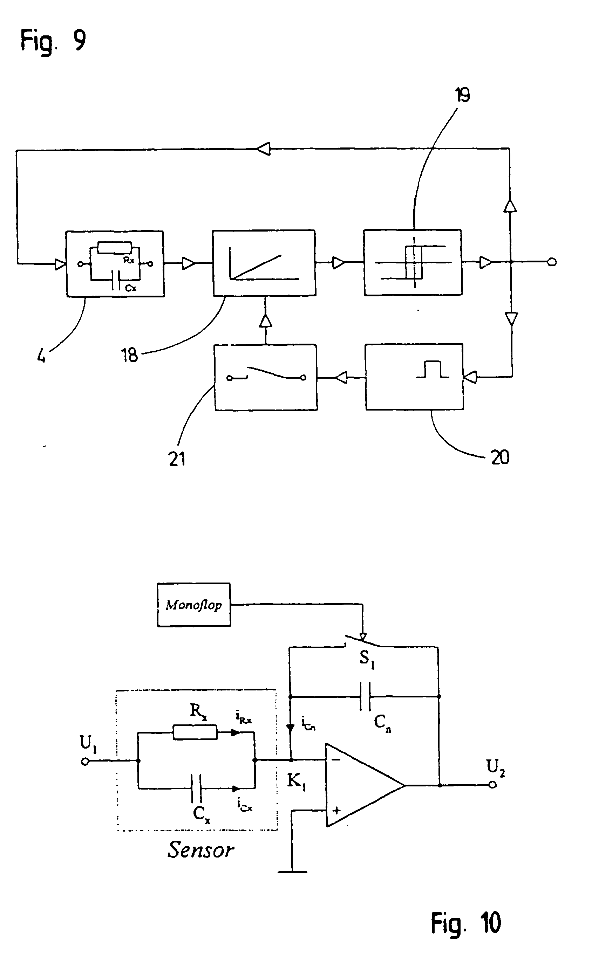

Bei dem in dem vereinfachten Blockschaltbild nach Fig. 9 dargestellten Messsystem zur Messung der spezifischen elektrischen Leitfähigkeit eines gering leitenden oder nichtleitenden Fluids wie Walzöl wird auf den Basissensor 4 ein Testsignal in Form einer getakteten Gleichspannung geschaltet, deren Frequenz vom Wert der Leitfähigkeit $\kappa$ abhängt. Die Ausgangsspannung des Basissensors 4 wird durch den Integrator 18 aufintegriert und das Ausgangssignal des Integrators 18 auf einen Schmitt-Trigger 19 geschaltet, dessen Ausgangssignal wiederum als Testsignal auf den Basissensor 4 geschaltet wird. Die Integration wird bei jeder Änderung des Testsignals auf null gesetzt.

Das Schaltungskonzept für das Messsystem zur Messung der Leitfähigkeit besteht im Kern aus einer astabilen Kippstufe mit dem Umkehrintegrator 18 und dem nichtinvertierenden Schmitt-Trigger 19, wobei der durch ein Monoflop 20 gesteuerte Schalter 21 den Integrator 18 während des Polaritätswechsels der Messspannung deaktiviert und auf die Anfangsbedingung null setzt.

Der bei dem Messsystem verwendete Integrator mit Operationsverstärker und dem Integrationskondensator $C_n$ ist für die Messgenauigkeit ausschlaggebend. Da der zu messende Widerstand $R_x$ bei sehr geringen Leitfähigkeiten eines Fluids Werte bis in den Terraohmbereich annehmen kann, sollte der eingesetzte Operationsverstärker einen hohen Eingangswiderstand besitzen. Die Offsetgrößen werden durch das erfindungsgemäße Messerverfahren eliminiert.

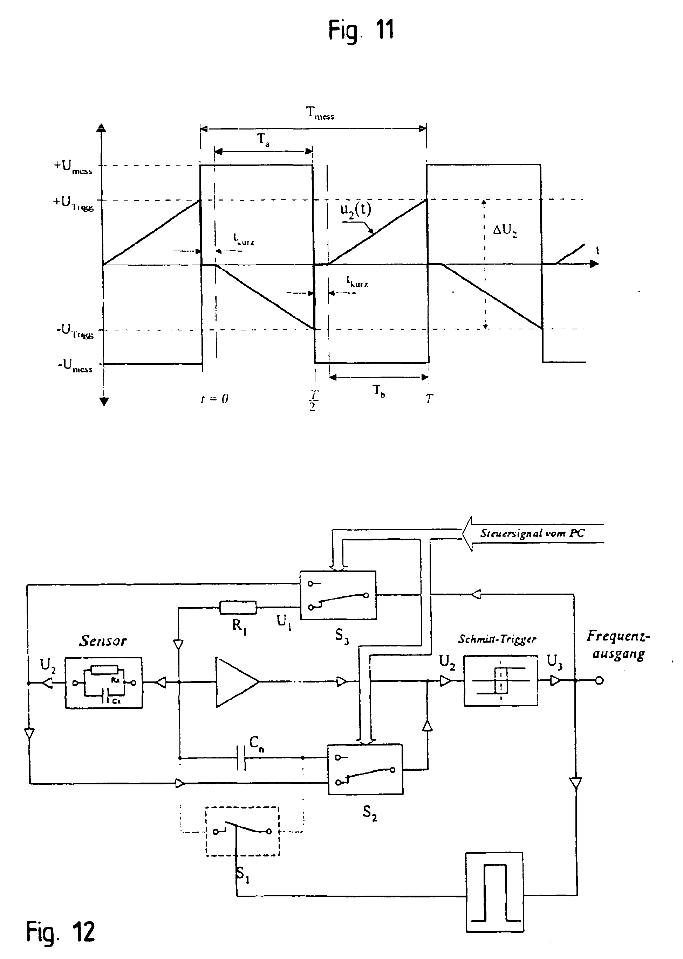

Fig. 10 zeigt den aktiven Integrator mit seiner Beschaltung und Fig. 11 das Diagramm der getakteten Eingangsspannung und der Ausgangsspannung des Integrators. Der Sensor, bestehend aus der Parallelschaltung von $R_x$ und $C_x$, bildet die Eingangsimpedanz. In der Rückkopplung befindet sich der Integrationskondensator $C_n$ und parallel dazu der über eine Monoflop gesteuerte Analogschalter $S_1$.

Wechselt die getaktete Gleichspannung $U_1$ ihr Vorzeichen, wird der Schalter $S_1$ geschlossen. Da sich der Integrator dann in direkter Gegenkopplung befindet, springt die Ausgangsspannung $U_2$ zu diesem Zeitpunkt auf null Volt, und der Integrationskondensator $C_n$ entlädt sich über den Schalter $S_1$. Die parasitäre Sensorkapazität $C_x$ wird während des Polaritätswechsels von $U_1$ lediglich umgeladen.

Hat die Messspannung wieder einen konstanten Wert erreicht, öffnet sich $S_1$ und der Integrationsprozess wird gestartet. Da $C_x$ nun bereits aufgeladen ist und damit entsprechend Gleichung (2) $i_c=0$ ist, wird nur noch der über den gesuchten Widerstand $R_x$ fließende Messstrom $i_{RX}$ aufintegriert.

Die Ausgangsfrequenz der Elektronik ist:

$$ f \; = \; \frac{\pi \cdot 1 \cdot R_3}{R_2 \cdot C_n \cdot \ln\left(\dfrac{r_a}{r_i}\right)} \; \cdot \; \kappa \qquad\qquad\qquad (3) $$

Die Frequenz ist gemäß Gleichung (3) linear von $\kappa$ abhängig. Die anderen Grö0en sind konstant. Sie beruhen auf den geometrischen Abmessungen des Sensors sowie auf dem Widerstandsverhältnis $R_3/R_2$, welches die Schaltschwellen des nicht näher beschriebenen Schmitt-Triggers bestimmt.

Die Berechnung des Integrators gemäß Fig. 10 zeigt, dass mit Kurzschließen des Integrationskondensators während des Polaritätswechsels von $U_{mess}$ die parasitäre Sensorkapazität eliminiert werden kann. Dazu muss der Schalter $S_1$ mit einem Steuerimpuls konstanter Länge $t_{kurz}$ angesteuert werden.

Fig. 12 veranschaulicht das Blockschaltbild eines Messsystems zur Messung der Leitfähigkeit und der Dielektrizitätszahl eines Fluids.

Durch die Messung der Dielektrizitätszahl kann der Wassergehalt beispielsweise von Transformatorölen, Bremsflüssigkeiten und Flugbenzin ermittelt werden. Bremsflüssigkeit kann Wasser aufnehmen, das in der Bremsflüssigkeit Dampfblasen bildet, die die Funktionstüchtigkeit der Bremsanlage in Kraftfahrzeugen beeinträchtigen. Bei Flugzeugen, die Temperaturen bis zu -40°C ausgesetzt sind, kann in der Bremsflüssigkeit enthaltenes Wasser Eiskristalle bilden, die Leitungen und Ventile der Bremsanlage verstopfen und dadurch die Bremsleitung vermindern, so dass bei der Landung der Flugzeuge eine

Unfallgefahr entstehen kann. Durch Wasser im Flugbenzin kann z.B. durch Vereisung oder Dampfblasenbildung die Leistung der Flugmotoren vermindert werden. Mithin kommt der Messung der Dielektrizitätszahl des Flugbenzins beim Betanken von Flugzeugen und der laufenden Messung der Dielektrizitätszahl der Bremsflüssigkeit der Bremsanlage von Flugzeugen zur Bestimmung des Wassergehaltes des Flugbenzins bzw. der Bremsflüssigkeit eine erhöhte sicherheitstechnische Bedeutung zu.

Die vorstehende Beschreibung zeigt, dass das erfindungsgemäße Messverfahren und die entsprechenden Messsysteme zur Messung der Leitfähigkeit und der Dielektrizitätszahl von gering leitenden und nichtleitenden Fluiden wie Walzöl, Schmierölen, Bremsflüssigkeiten und Flugbenzin auf vielen technischen Anwendungsgebieten in der Zukunft eine große Bedeutung erlangen werden.

**Patentansprüche**

1.    Verfahren zur Online-Messung physikalischer Größen von gering leitenden und nichtleitenden Fluiden, wobei sich das Fluid zwischen zwei elektrisch leitenden Elektroden eines Basissensors (4) befindet, **gekennzeichnet durch** eine Messung der elektrischen Leitfähigkeit κ zur Regelung einer elektrostatische Auf- und Entladungen verhindernden Mindestleitfähigkeit von Fluiden **durch** eine geregelte Einleitung von Leitfähigkeitsadditiven und eine Messung der zeitlichen Änderung der Leitfähigkeit zur Bestimmung des Feinstverschmutzungseintrags und des Verunreinigungsgrades insbesondere **durch** Partikel, wobei auf den Basissensor (4) ein Testsignal in Form einer getakteten Gleichspannung oder eines getakteten Gleichstroms geschaltet wird, mit einer vom Wert der Leitfähigkeit κ abhängigen Frequenz der sich einstellende Ausgangsstrom oder die Ausgangsspannung mittels eines Integrators (18) aufintegriert wird, dessen Ausgangssignal auf einen Schmitt-Trigger (19) geschaltet wird, dessen Ausgangssignal wiederum als Testsignal auf den Basissensor (4) geschaltet wird, und wobei die Integration bei jeder Änderung des Testsignals (Flanken) auf null gesetzt wird.

**2.** Verfahren nach Anspruch 1, **dadurch gekennzeichnet, dass** durch eine Unterschreitung einer vorgegebenen Mindestleitfähigkeit die Gefahr einer elektrostatischen Aufladung und einer unkontrollierten Entladung erkannt wird.

**3.** Verfahren nach Anspruch 1, **gekennzeichnet durch** eine Differenzmessung der Leitfähigkeit vor und hinter einem Filter zur Prüfung der Funktion und Güte eines Filtervorgangs und gegebenenfalls zur Regelung des Filtervorgangs **durch** eine geregelte Zuführung von Filterhilfsmitteln.

**4.** Verwendung der Verfahren nach den Ansprüchen 1 bis 3 zur Untersuchung von Ölen wie Walzöl, Schmieröl und Hydrauliköl.

**5.** Verwendung der Verfahren nach den Ansprüchen 1 bis 3 zur Untersuchung von Bremsflüssigkeiten.

**6.** Verwendung der Verfahren nach den Ansprüchen 1 bis 3 zur Untersuchung von Kraftstoffen.

**7.** Messsystem zur Online-Messung der elektrischen Leitfähigkeit κ von gering leitenden und nichtleitenden Fluiden, wobei sich das Fluid zwischen zwei elektrisch leitenden Elektroden eines Basissensors (4) befindet, **dadurch gekennzeichnet, dass** der Basissensor (4) eine als elektrisch leitender Rohrabschnitt (9a) ausgebildete Außenelektrode (9) aufweist, in dem mindestens eine elektrisch leitende, vom Fluid umströmte Innenelektrode (10) angeordnet ist, die aus mindestens zwei voneinander isolierten, elektrisch leitenden Teilen in Form einer Nutzelektrode (10a) und einer Schirmelektrode (10b, 10c) besteht, die zur Durchführung von Anschlussleitungen zumindest teilweise hohl ausgebildet sind, wobei die für die Messung bestimmte Nutzelektrode (10a) der Innenelektrode (10) keinen direkten mechanischen Kontakt zu Halterungen (12) der Innenelektrode (10) besitzt und auf die Schirmelektrode (10b, 10c) der Innenelektrode (10) gleiche, jedoch elektrisch unabhängige Spannungen wie auf die für die Messung bestimmte Nutzelektrode (10a) geschaltet werden, wobei die Schirmelektrode (10b, 10c) zur Erzeugung einer geeigneten elektrischen Feldverteilung bestimmt ist und die Funktion von Halterungen übernimmt, wobei das Messsystem so eingerichtet ist, dass auf den Basissensor (4) ein Testsignal in Form einer getakteten Gleichspannung geschaltet wird, deren Frequenz vom Wert der Leitfähigkeit κ abhängt, die Ausgangsspannung des Basissensors (4) durch einen Integrator (18) aufintegriert und das Ausgangssignal des Integrators auf einen Schmitt-Trigger (19) geschaltet wird, dessen Ausgangssignal wiederum als Testsignal auf den Basissensor (4) geschaltet wird, wobei die Integration bei jeder Änderung des Testsignals auf null gesetzt wird.

**8.** Messsystem nach Anspruch 7, **dadurch gekennzeichnet, dass** die Innenelektrode (10) des Basissensors (4) für Viskositätsmessungen rotierbar ist.

**9.** Messsystem nach Anspruch 7, **dadurch gekennzeichnet, dass** das Sensorsystem als planare Elektrodenanordnung mit nebeneinander liegenden oder konzentrisch angeordneten Elektroden ausgebildet ist, wobei neben den Nutzelektroden eine weitere Elektrode zur Schirmung und Formung des elektrischen Feldes dienen.

## Claims

**1.** Method for on-line measurement of physical quantities of poorly conductive and non-conductive fluids, wherein the fluid is located between two electrically conductive electrodes of a base sensor (4), **characterised by** a measurement of the electrical conductivity κ to control a minimum conductivity of fluids that prevents electrostatic charges and discharges by a controlled introduction of conductivity additives and a measurement of the time change rate of the conductivity to determine the superfine contamination inclusion and the degree of contamination in particular as a result of particles, wherein a test signal in the form of a clocked d.c. voltage or a clocked d.c. current is switched to the base sensor (4), the adjusting output current or the output voltage is integrated with a frequency dependent on the value of the conductivity $\kappa$ by means of an integrator (18), the output signal of which is switched to a Schmitt trigger (19), the output signal of which is in turn switched as test signal to the base sensor (4), and wherein the integration is set to zero upon each change in the test signal (pulse edges).

2. Method according to claim 1, **characterised in that** the risk of an electrostatic charge and an uncontrolled discharge is detected by a predefined minimum conductivity not being reached.

3. Method according to claim 1, **characterised by** a differential measurement of the conductivity in front of and behind a filter to test the function and quality of a filtering process and, if necessary, for controlling the filtering process by a controlled supply of filter aids.

4. Use of the methods according to claims 1 to 3 for examining oils such as roll oil, lubricating oil and hydraulic oil.

5. Use of the methods according to claims 1 to 3 for examining brake fluids.

6. Use of the methods according to claims 1 to 3 for examining fuels.

7. Measuring system for on-line measurement of the electrical conductivity $\kappa$ of poorly conductive and non-conductive fluids, wherein the fluid is located between two electrically conductive electrodes of a base sensor (4), **characterised in that** the base sensor (4) has an external electrode (9) configured as an electrical conductive pipe section (9a), in which at least one electrically conductive internal electrode (10) is arranged, which has fluid flowing around it and comprises at least two mutually insulated electrically conductive parts in the form of an effective electrode (10a) and a shield electrode (10b, 10c), which are configured to be at least partially hollow for the passage of connection leads, wherein the effective electrode (10a) of the internal electrode (10) intended for the measurement has no direct mechanical contact with mountings (12) of the internal electrode (10) and the same, but electrically independent voltages are connected to the shield electrode (10b, 10c) of the internal electrode (10) as to the effective electrode (10a) intended for the measurement, wherein the shield electrode (10b, 10c) is intended for generation of a suitable electric field distribution and takes over the function of mountings, wherein the measuring system is arranged such that a test signal in the form of a clocked d.c. voltage is switched to the base sensor (4), the frequency of which is dependent on the value of the conductivity $\kappa$, the output voltage of the base sensor (4) is integrated by means of an integrator (18) and the output signal of which is switched to a Schmitt trigger (19), the output signal of which is in turn switched as test signal to the base sensor (4), wherein the integration is set to zero upon each change in the test signal.

8. Measuring system according to claim 7, **characterised in that** the internal electrode (10) of the base sensor (4) is rotatable for viscosity measurements.

9. Measuring system according to claim 7, **characterised in that** the sensor system is configured as a plane electrode arrangement with adjacent or concentrically arranged electrodes, wherein besides the effective electrodes a further electrode serves to shield and form the electric field.

**Revendications**

1. Procédé de mesure en ligne de grandeurs physiques de fluides peu conducteurs et non conducteurs, le fluide se trouvant entre deux électrodes conductrices de l'électricité d'un capteur (4) de base, **caractérisé par** une mesure de la conductivité $\kappa$ électrique pour la régulation d'une conductivité minimum de fluide empêchant des charges et des décharges électrostatiques par une introduction régulée d'additifs de conductivité et une mesure de la variation en fonction du temps de la conductivité pour la détermination de l'apport de pollution le plus petit et du degré de pollution, notamment par des particules, dans lequel on applique au capteur (4) de base un signal de test, sous la forme d'une tension continue cadencée ou d'un courant continu cadencé, on intègre au moyen d'un intégrateur (18), à une fréquence du courant de sortie ou de la tension de sortie qui s'établit, qui dépend de la valeur de la conductivité $\kappa$, le signal de sortie de l'intégrateur étant appliqué à un déclencheur (19) de Schmitt, dont le signal de sortie est appliqué à nouveau, en tant que signal de test, au capteur (4) de base et l'intégration étant remise à zéro à chaque modification du signal de test (fronts).

2. Procédé suivant la revendication 1, **caractérisé en ce que** l'on reconnaît le danger d'une charge électrostatique et d'une décharge incontrôlées par le fait que l'on passe en dessous d'une conductivité minimum prescrite.

3. Procédé suivant la revendication 1, **caractérisé par** une mesure de différence de la conductivité avant et après un filtre pour contrôler le fonctionnement et la qualité d'une opération de filtration, le cas échéant pour réguler l'opération de filtration par un apport régulé d'adjuvants de filtration.

4. Utilisation du procédé suivant les revendications 1 à 3 pour étudier des huiles comme de l'huile de laminage, de l'huile de lubrification et de l'huile hydraulique.

5. Utilisation du procédé suivant les revendications 1 à 3 pour étudier les liquides de freinage.

6. Utilisation du procédé suivant les revendications 1 à 3 pour étudier les carburants.

7. Système de mesure en ligne de la conductivité $\kappa$ électrique de fluides peu conducteurs et non conducteurs, le fluide se trouvant entre deux électrodes conductrices de l'électricité d'un capteur (4) de base, **caractérisé en ce que** le capteur (4) de base a une électrode (9) extérieure constituée sous la forme d'un tronçon (9a) de tube conducteur de l'électricité, dans lequel est disposée au moins une électrode (10) intérieure conductrice de l'électricité devant laquelle passe du fluide et qui est constituée d'au moins deux parties conductrices de l'électricité et isolées l'une de l'autre sous la forme d'une électrode (10a) utile et d'une électrode (10b, 10c) de protection, qui sont creuses au moins en partie pour le passage de lignes de connexion, l'électrode (10a) utile, destinée à la mesure, de l'électrode (10) intérieure n'ayant pas de contact mécanique direct avec des fixations (12) de l'électrode (10) intérieure et il est appliqué à l'électrode (10b, 10c) de protection de l'électrode (10) intérieure de mêmes tensions mais indépendantes électriquement qu'à l'électrode (10a) utile destinée à la mesure, l'électrode (10b, 10c) de protection étant destinée à produire une répartition du champ électrique approprié et à prendre en charge la fonction de fixations, le système de mesure étant tel qu'il est appliqué au capteur (4) de base un signal de test, sous la forme d'une tension continue cadencée, dont la fréquence dépend de la valeur de la conductivité $\kappa$, la tension de sortie du capteur (4) de base étant intégrée par un intégrateur (18) et le signal de sortie de l'intégrateur étant appliqué à un déclencheur (19) de Schmitt, dont le signal de sortie est appliqué à nouveau, en tant que signal de test, au capteur (4) de base, l'intégration étant remise à zéro à chaque variation du signal de test.

8. Système de mesure suivant la revendication 7, **caractérisé en ce que** l'électrode (10) intérieure du capteur (4) de base peut tourner pour des mesures de viscosité.

9. Système de mesure suivant la revendication 7, **caractérisé en ce que** le système de capteur est constitué sous la forme d'un dispositif d'électrodes plan ayant des électrodes côte à côte ou concentriques, une autre électrode à côté de l'électrode utile servant à protéger et à former le champ électrique.

**Fig. 1**

**Fig. 2**

Fig. 3

Fig. 4

Fig. 5

Fig. 6

## Fig. 7

*Sensor*

## Fig. 8

Fig. 9

Fig. 10

EP 0 819 938 B1

Fig. 11

Fig. 12

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5124654 A
- US 5399979 A
- SU 1044893 A

**In der Beschreibung aufgeführte Nicht-Patentliteratur**

- **OEHME, F.** Chemische Sensoren. 1991